# EUROPEAN PATENT SPECIFICATION

(11) **EP 3 049 102 B1**
(45) Date of publication and mention of the grant of the patent: **04.03.2020**
(21) Application number: 14781676.3
(22) Date of filing: 04.09.2014
(51) Int. Cl.: A61K 38/40, A61K 9/16, A61K 9/20, A61P 1/04

(54) **LACTOFERRIN FOR THE TREATMENT OF IBD ASSOCIATED WITH BACTERIAL INVASION**
LACTOFERRIN FÜR DIE BEHANDLUNG VON CHRONISCH ENTZÜNDLICHER DARMERKRANKUNG ASSOZIIERT MIT BAKTERIELLER INVASION
LACTOFERRIN POUR LE TRAITEMENT DU SYNDROME ABDOMINAL INFLAMMATOIRE ASSOCIE A UNE INVASION BACTERIENNE

(30) Priority: 25.09.2013 IT MI20131578
(43) Date of publication of application: 03.08.2016
(73) Proprietor: Giellepi S.p.A., 20123 Milano (IT)
(72) Inventor: TERRUZZI, Carlo, I-20836 Briosco (MB) (IT); TERRUZZI, Fabio, I-20836 Briosco (MB) (IT)
(74) Representative: Montelatici, Linda Anna
(86) International application number: PCT/IB2014/064255
(87) International publication number: WO 2015/044809

(56) References cited:
- EP-A1- 1 350 519
- WO-A2-2007/038623
- US-A1- 2012 171 163
- LI LI ET AL: "Determination of the effects of lactoferrin in a preclinical mouse model of experimental colitis.", MOLECULAR MEDICINE REPORTS OCT 2013, vol. 8, no. 4, 13 August 2013 (2013-08-13) , pages 1125-1129, XP009174514, ISSN: 1791-3004
- PINETON DE CHAMBRUN GUILLAUME ET AL: "Pathogenic agents in inflammatory bowel diseases.", CURRENT OPINION IN GASTROENTEROLOGY JUL 2008, vol. 24, no. 4, July 2008 (2008-07), pages 440-447, XP009174481, ISSN: 1531-7056
- JENNIFER R. BAILEY ET AL: "Identification and Characterisation of an Iron-Responsive Candidate Probiotic", PLOS ONE, vol. 6, no. 10, 19 October 2011 (2011-10-19), page e26507, XP055048364, ISSN: 1932-6203, DOI: 10.1371/journal.pone.0026507
- S MIEHLKE ET AL: "Direct activity of recombinant human lactoferrin against Helicobacter pylori", J. CLIN. MICROBIOL, vol. 34, no. 10, 1 January 1996 (1996-01-01), pages 2593-2594, XP055089863,
- TOGAWA JUN-ICHI ET AL: "Oral administration of lactoferrin reduces colitis in rats via modulation of the immune system and correction of cytokine imbalance", JOURNAL OF GASTROENTEROLOGY AND HEPATOLOGY, WILEY-BLACKWELL PUBLISHING ASIA, AU, vol. 17, no. 12, 1 December 2002 (2002-12-01), pages 1291-1298, XP009100413, ISSN: 0815-9319, DOI: 10.1046/J.1440-1746.2002.02868.X
- EMILIE VAZEILLE ET AL: "Role of Meprins to Protect Ileal Mucosa of Crohn's Disease Patients from Colonization by Adherent-Invasive E. coli", PLOS ONE, vol. 6, no. 6, 16 June 2011 (2011-06-16), page e21199, XP055089864, DOI: 10.1371/journal.pone.0021199

## Description

The present invention relates to the treatments of inflammatory bowel diseases associated with Adherent-invasive Escherichia coli (AIEC) invasion, and in particular it relates to a formulation for use in such treatments.

Crohn's disease (CD), Ulcerative Colitis (UC) and IBDs of infectious origin, are multifactorial illnesses of often unknown aetiology. Deregulation of the innate and adaptive immune system directed against luminal bacteria or their products and inappropriate immune responses to organisms in the intestine that normally do not elicit a response are immune factors characteristic of CD, UC, and other IBDs.

According to the current understanding, microorganisms are taking advantage of the weakened mucosal layer of patients suffering from inflammatory bowel diseases and inability to clear bacteria from the intestinal walls, thus causing the diseases' symptoms.

Some studies have shown that adherent-invasive Escherichia coli (AIEC), are much more prevalent in CD patients than in controls.

Other studies, such as that published in Curr Opin Gastroenterol. 2007; 23(1):16-20 "Adherent-Invasive Escherichia coli and Crohn's Disease" by Nicolas Bamich, Arlette Darfeuille-Michaud, indicated that as the infection cycle of adherent-invasive E. coli could depend upon the AIEC ability to colonize the gastrointestinal tract of genetically predisposed Crohn's disease patients, antibiotics which could eradicate the bacteria, or probiotics which could substitute them in the gastrointestinal tract, could be of therapeutic value in ileal Crohn's disease.

Adherent-Invasive Escherichia coli encompass a subgroup of E. coli spp. named from their characteristic capability to adhere to intestinal cells, to invade the infected eukaryotic cells, and to replicate in epithelial cells and macrophages, causing intestinal diseases in humans. LF82, an E. coli strain (serotype O83:H1) originally isolated from a patient with Crohn disease represents the prototype AIEC strain, as described in Darfeuille-Michaud A., Neut C., Barnich N., Lederman E., Di Martino P., Desreumaux P. et al. "Presence of adherent Escherichia coli strains in ileal mucosa of patients with Crohn's disease" Gastroenterology, 115 (1998), pp. 1405-1413.

Lactoferrin (Lf), a member of the tranferrin family of iron-binding glycoproteins constitutes one of the major antimicrobial systems in milk and other exocrine secretions. Biological properties reported for Lf include antimicrobial activity against a wide range of pathogenic bacteria, fungi, protozoa and virus, as well as anti-inflammatory, antitumour and immuno-modulatory activities, as described in Steijns, J.M. and Van Hooijdonk, A.C.M. "Occurrence, structure, biochemical properties and technological characteristics of lactoferrin" Br J Nutr 2000; 84, 511-517; and Superti, F.; Berlutti, F.; Paesano, R.; Valenti, P. "Structure and activity of lactoferrin - A multi functional protective agent for human health". Iron Metabolism and Disease; Fuchs, H., Ed.; Research Signpost: Kerala, India, 2008; pp. 1-32.

Generally, treatment of acute inflammatory bowel disease states comprises the use of medications, for example antibiotic drugs, to treat any infection and anti-inflammatory drugs and corticosteroids to reduce inflammation. However, prolonged use of antibiotics, anti-inflammatory drugs and corticosteroids has significant side-effects so that it has to be avoided.

WO2007038623 discloses the use of a lactoferrin oral formulation for treatment, of IBD and Crohn's disease associated infection by intestinal pathogens such as C. difficile infection.

EP1350519 discloses a controlled release oral composition comprising lactoferrin used for treating a number of conditions, eg IBD.

Therefore, object of the present invention is to provide a formulation for use in the treatment of patients affected by inflammatory bowel disease associated with Adherent-invasive Escherichia coli (AIEC) invasion, which provides no significant side-effects.

This object has been achieved by means a modified release formulation comprising lactoferrin for use in the treatment of inflammatory bowel disease associated with Adherent-invasive Escherichia coli (AIEC) invasion according to claim 1, while other features are disclosed in the remaining claims.

According to the present invention, it has been discovered that lactoferrin, particularly bovine lactoferrin, exerts strong inhibitory effects on AIEC invasion of intestinal epithelial cells and can be used in the treatment of inflammatory bowel diseases and for maintaining remission in patients harbouring pathogenic AIEC colonizing early and chronic ileal lesions.

As a matter of fact, tests carried out by the inventors show that a lactoferrin pepsin hydrolysate is able to prevent AIEC adhesion and internalization into intestinal cells while it is unable to reduce inflammation. On the contrary, lactoferrin surprisingly strongly inhibits AIEC invasion of intestinal cells and reduces inflammation.

According to the invention it was further found that, at non-cytotoxic and non-bactericidal concentration, lactoferrin treatment had no effect on bacterial adhesion whereas was able to prevent the invasion of intestinal cells by the prototype AIEC strain LF82 in a dose-dependent manner.

Therefore the modified release formulations comprising lactoferrin may be used in the treatment of chronic inflammatory conditions associated with an invasion of AIEC.

In particular, the use of bLf, being free from negative side effects, could be used in the treatment of Crohn's disease and other inflammatory bowel diseases associated with an AIEC invasion, and in the maintenance of the remission state of the patients.

Following the above mentioned findings, the modified release formulations comprising lactoferrin or preferably bovine lactoferrin according to the present disclosure have been obtained by a careful and purposive selection of the known modified release systems and of the substances therein employed.

As a matter of fact, the lactoferrin modified release formulations according to the present disclosure have a localized activity in different gastrointestinal tract or a systemic activity, and a controlled release from the early stages of administration. In addition, homogeneity of release in time is ensured and at the same it is allowed that, once a part of the active principle has been made available, it is immediately active either topically or at systemic level, due to its state of microemulsion, solubilization or complexation.

According to the present invention, there is provided an oral formulation for modified release dosage comprising lactoferrin for use in the treatment of inflammatory bowel diseases such as Crohn's disease, ulcerative colitis, and intestinal disease due to Adherent-invasive Escherichia coli (AIEC) invasion, most particularly for use in the treatment of inflammatory bowel diseases due to LF82 Escherichia coli invasion.

In particular, modified release dosage means a delayed release that allows to obtain the advantage of a reduced frequency of administration and a release of the active substance in particular sites of the gastrointestinal tract.

The oral formulation for modified release dosage for use according to the present invention comprise lactoferrin dissolved and/or dispersed and/or embedded in a matrix and an outer coating, wherein said matrix comprises:
- substances selected in the group of: lipid substances, hydrophilic substances, amphiphilic substances and mixtures thereof; and
- optionally, pharmaceutically acceptable excipients intended to facilitate the feasibility of the pharmaceutical form.

As lactoferrin in the oral formulation according to the present invention bovine lactoferrin may be used. The amount of lactoferrin in the oral formulations according to the present disclosure is preferably suitable to allow the administration of lactoferrin in a daily dosage of between 50 mg and 250 mg; preferably between 100 mg and 200 mg, in one or more daily administrations.

Said outer coating is preferably formed of materials provided with gastric solubility property or gastric resistance property and/or with property of release in specific intestinal pH (pH dependent release property) or property of pH independent delayed release.

Suitable materials for said outer coating of the oral formulations according to the present disclosure comprise acetate cellulose phthalate, methacrylic acetate, methacrylic acid polymers, shellac, ethylcellulose, alginic acids and combination thereof; preferably gastroresistant coating agents selected in the group consisting of Shellac, Polymethacrylates, Cellulose Acetophtalate, Alginate Derivatives can be mentioned. Preferable, aqueous Shellac (for example the product Aquagold® by Harke) and combinations thereof, or a combination of ethylcellulose and alginic acid salts marketed as Nutrateric® nutritional enteric coating system by Colorcon Inc. of Westpoint, Pa may be used.

The lipid substances that can be used in the matrix of the oral composition according to the disclosure include fatty alcohols, fatty acids, triglycerides, waxes. Preferably, said lipid substances are selected in the group consisting of palmitic acid, cetyl alcohol, cetostearyl alcohol, carnauba wax, stearic acid.

The hydrophilic substances that can be used in the matrix of the oral composition according to the disclosure comprise hydrogels, i.e. substances that switching from the anhydrous to the hydrate state show a phenomenon of molecular relaxation, characterized by a substantial increase in volume of the system and by an increase in size and weight following the coordination of a large number of water molecules by the polar groups present in the polymer chain. The hydrogels that can be used according to the invention, are selected in the group of polymers or copolymers of acrylic or methacrylic acid, alkyl vinyl ether polymers, hydroxyalkylcelluloses, carboxyalkylcelluloses, polysaccharides, alginates, pectins, starches, natural and synthetic gums, polycarbophil and mixtures thereof. Preferably, said hydrophilic substances comprise hydroxypropylmethylcellulose (for example sold under the tradenames Methocel® K4M, K15M, K100M, 100LV), carboxymethycellulose (for example sold under the tradename Blanose®), hydroypropylcellulose (for example sold under the tradenames Klucel® EF, LF, JF, GF, MF, HF), microcrystalline cellulose, starches such as corn starch and lactose.

The amphiphilic matrix substances that can be used in the formulations according to the invention comprise lecithin, phosphatidylcholine, phosphatidyl-ethanolamine, glycol alkyl ethers such as diethylene glycol monomethyl ether, macrogolglycerides, polyethylene glycol hydroxystearates, waxes, sodium laurylsulfate, sodium dodecylsulfate, polysorbates, cholic acid, poloxamer, sodium sulphosuccinate, sodium laurylsarcosinate and mixtures thereof. Preferably, said amphiphilic substances comprise lauroyl polyoxyglycerides (for example the product marketed under the tradename Gelucire® 44/14 or Labrafil®M2130Cs), hydrogenated coconut oil, PEG 1500 esters, stearoyl polyoxyglycerides (for example the product marketed under the tradename Gelucire® 50/13, hydrogenated palm oil, lechitin (for example the products marketed under the tradename Epikuron®, Phosal®, Phospholipon® 100 H, Lipoid®).

Said pharmaceutically acceptable excipients that are intended to facilitate the feasibility of the pharmaceutical form may be selected among gliding agents, diluents, anti adherent agents, lubricants, disintegrants, free flowing agents, stabilizers.

According to an alternative embodiment, the oral formulation for use according to the invention is in the form of a reservoir system wherein lactoferrin is dissolved and/or dispersed and/or embedded in a reservoir core that is coated by cellulose membranes semipermeable to water, preferably ethylcellulose membranes.

The reservoir cores can be considered containers of lactoferrin that are properly coated with semipermeable membranes made of cellulose derivatives such as ethylcellulose, methylcellulose, methyl ethyl cellulose, hydroxypropyl methylcellulose.

Other optional components of the oral compositions according to the present invention are film forming excipients and granulation excipients can in turn be selected among the gastrosoluble coating agents, gastroresistant coating agents, pH dependent coating agents, pH independent coating agents provided with site specific release properties and include cellulose acetate phtalate, methacrylic acid polymers, shellac, ethylcellulose, alginic acids.

In addition, the formulations for use according to the present invention may comprise other active substances such as anti-inflammatory drugs, mesalamine, mesalamine derivatives, corticosteroids, azathioprine, cyclosporine, monoclonal antibodies.

The oral formulations for use according to the present invention can be produced by a manufacturing processes selected among those normally used in pharmaceutical manufacturing areas such as direct compression process, wet granulation process, compaction/dry granulation process, and a process comprising granulation by fusion and loading / co-grinding and final filling of powder.

In particular, the oral formulations for use according to the invention may be obtained by a method consisting of the following stages:
a) mixing sieved lactoferrin with matrix substances selected among lipid substances, hydrophilic substances, amphiphilic substances;
b) optionally, adding any other active substances;
c) carrying out a step selected in the group consisting of wet granulation, dry granulation, direct filling, co-grinding, melt granulation;
d) optionally, adding other pharmaceutically acceptable excipients intended to facilitate the feasibility of the pharmaceutical form, such as: gliding agents, diluents, anti adherent agents, lubricants, disintegrants, to obtain the solid form in powder and/or granules and/or minitablets and/or microgranules with good free flowing and compressibility properties;
e) film coating by means of gastrosoluble or gastroresistant excipients or pH dependent and/or pH-independent excipients capable of delaying the release of lactoferrin, in order to obtain an external coating providing site specific release in the gastrointestinal tract.

According to the disclosure, for the preparation of a monolithic matrix system a lipid or amphiphilic or hydrophilic matrix containing the lactoferrin is prepared, then various functional excipients are added to dilute and make feasible the product through the suitable pharmaceutical processes. The ratio between the amounts of drug and matrix substances with respect to the excipients that are added in this step does not normally exceed 1:4; the optimal quantity of drug and matrix substances is between 0.1% and 20% by weight of the amount of excipients.

To this mixture, it is possible to add diluents in an amount up to 50% by weight, lubricants in an amount of 0.5-3% by weight, gliding agents in an amount of 0.5-3% by weight, and disintegrants in an amount of 0.1 - 40% by weight, all percentages being referred to the final unit dosage form weight.

An alternative pharmaceutical form of lactoferrin consist in reservoir systems. To prepare a lactoferrin reservoir system, a core containing pharmaceutical multi particulate or monolithic lactoferrin is prepared by loading lactoferrin on inert excipients such as diluents, gliding agents, lubricants. The weight ratio between lactoferrin and diluent agents may be for example 1:1; the weight ratio between lactoferrin and gliding agents may be for example 1:0,5; the weight ratio between lactoferrin and lubricants may be for example 1:0,5. The resulting core is then coated with a semi permeable membrane that regulates the lag time and release time. The membrane may be formed of cellulose polymers and derivatives thereof, and the coating provides an increase of the weight of the system from 0.5% to 30%.

In terms of dissolution characteristics, the contact of these formulations with water or aqueous fluids causes the modified and/or delayed and/or slowed and/or the site specific release of the active substance which is immediately dispersed, solubilized in the so formulated system. The excipients and polymers present in the structure rule the wettability of the system and the homogeneous solubilization of lactoferrin in narrow release ranges, thus favoring its localized activity and/or a continuous and gradual absorption or the gradual topical release in the gastrointestinal tract.

Further advantages and features of the lactoferrin formulations for use according to the present invention will become clear to those skilled in the art from the following examples with reference to the attached figures, wherein:
- figure 1 is a graph showing the effect of bLf and bLfH (1 mg/ml) on the adhesion ability of LF82 to epithelial cells;
- figure 2 is a graph showing the effect of bLf and bLfH (1 mg/ml) on the invasion ability of LF82 to epithelial cells;
- figures 3A, B and C are graphs showing the effect of bLf (1 mg/ml) on TNF alpha, IL 8 and IL6 mRNA expression in epithelial cells;
- figures 4A and B are graphs showing the effect of bLf on TNF-alpha, IL-8 and IL-6 mRNA expression in cultured mucosal explants infected with LF82 for 6 hours (A) and 24 hours (B); and
- figure 5 is a graph showing the effect of the addition of bLf on the pro-inflammatory cytokine mRNA expression, in particular TNF-alpha, in cells treated with INF-gamma.

### Materials and Methods

The adherent, invasive E. coli strains utilized in this study was LF82 (ileal Crohn's strain, kindly provided by Arlette Darfeuille-Michaud, Clermont Université, Université d'Auvergne, Clermont-Ferrand, France).

LF82 was cultured in MacConkey agar plates for 24 hrs at 37°C and then subcultured in Luria Bertani Broth (LB, Oxoid) with overnight incubation in air at 37°C. Before infection of cells, bacteria were washed and re-suspended in cell culture medium at the suitable concentrations.

### Cell line cultures

Caco-2 (human colorectal adenocarcinoma) cells were obtained from the American Type Culture Collection (ATCC, Rockville, MA). Cells were grown at 37°C in a humidified atmosphere with 5% CO₂ in Dulbecco's minimum essential medium (DMEM, Gibco) supplemented with 10% inactivated fetal calf serum (FCS, Euroclone) and 2mM L-glutamine.

### Organ culture

The study was approved by the ethical committee of the University Hospital Umberto I where patients were admitted. For each patient informed consent from parents was obtained. After written consent, CD subject biopsies were obtained from intestinal tissue macroscopically not involved in chronic inflammation processes. For organ culture specimens were immediately placed in Trowell T8 medium and treated as described below.

Mucosal biopsy specimens were placed on iron grids with the mucosal face upward in the central well of an organ culture dish (Falcon, Becton Dickinson, NJ, USA) containing Trowell T8 medium and NCTC-135 medium (ratio 3:1) (Biowest, Miami, FL, USA) supplemented with 10% FCS (Euroclone). Dishes were placed in a modular incubator chamber (MP Biomedicals, Aurora, Ohio, USA) at high oxygen saturation (95%) and incubated at 37°C.

### Chemicals

Bovine lactoferrin (bLf) and its pepsin hydrolysate (bLfH), produced by Morinaga Milk Industry Co., Ltd. were dissolved in PBS at the concentration of 80 mg/ml.

### Cytotoxicity assay

To establish the maximal non-cytotoxic dose of bLf or bLf-hydrolysate, serial dilutions of each preparation were incubated at 37°C with the different cell lines grown in the different growth media at 37°C in a humidified 5% CO2 incubator. After 24, 48, and 72 h, the following parameters will be evaluated: cell enumeration, morphology and viability after dispersion into individual cells with trypsin.

### Antibacterial activity

The minimal inhibitory concentrations (MIC) of bLf and bLfh were determined by broth microdilution methods. Tests were performed with LF82 cells previously grown up to the exponential growth phase in Mueller-Hinton broth at a final concentration of 5 x 105 CFU/ml.

One hundred microliters of bacteria were added to the wells of a 96-well plate with 100µl of different bLf or bLfH concentrations (serial two-fold dilutions ranging from 0.08 to 40 mg/ml). The MIC was defined as the lowest protein or hydrolysate concentration that caused a complete inhibition of bacterial growth after 24 hours incubation at 37°C.

To determine the minimum bactericidal concentration (MBC), a volume of 100 µl was taken from the wells where no growth has been detected, spread on TSA plates and incubated at 37°C for 24 h. MBC will be defined as the concentration at which there is >99.9% (3 log) decrease in viable cells.

### Inhibition of LF82 adhesion to Caco-2 cells

Caco-2 cell monolayers were prepared in 24-well tissue culture plates (Falcon).

Before the adhesion test, cells were incubated at 37°C in DMEM supplemented with 10% FCS in presence or absence of bLf or bLfH (1mg/ml). After 2 hrs, cells were infected with LF82 at a multiplicity of infection (MOI) of 1 or 10 bacteria per cell. After 3h incubation at 37°C, the cells were washed 3 times and lysed with 0.1% TritonX-100 and the number of CFUs was determined by plating. Bacterial adhesion in the different experimental conditions was defined as the percentage of attached bacteria in comparison with adhesion of untreated bacteria taken as 100%. All assays were performed in triplicate.

### Inhibition of LF82 invasion in Caco-2 cells

To determine bacterial invasion, Caco-2 cell monolayers were infected with 1 or 10 bacteria per cell. After 3h incubation at 37°C, the cells were washed 3 times and incubated for additional 2h in medium supplemented with 0.1mg/ml gentamicin to kill extracellular bacteria. The inhibiting activity of 1mg/ml bLf or bLfH was assessed by incubating the cells 2 hrs before and during the infection, in presence or absence of proteins. Then monolayers were washed and lysed by adding deionized water containing 0.1 % Triton X-100 for 5 min to release internalized bacteria. All assays were performed in triplicate. Bacterial invasion in the different experimental conditions was defined as the percentage of the intracellular bacteria that are culturable after gentamicin treatment and cell lysis in comparison with untreated bacteria taken as 100%.

### Cytokine analysis in Caco-2 infected cells

Caco-2 cells were incubated absence or in presence of bLf of bLfH for 2hrs before and during infection (3 hrs at 37°C) with LF82 (MOIs 1 or 10). After infection, culture supernatants were collected, cleared from any cells and pathogens by centrifugation, and kept frozen at - 80° C until use. Supernatants and cell extracts were processed for cytokines (IL-6, IL-8, and TNF-alpha) by quantitative RealTime-PCR.

### Cytokine analysis in infected organ cultures

Organ cultures were incubated absence or in presence of bLf for before (2 hrs at 37°C) and during infection with LF82 (108 CFU/mL). Dishes were then placed in a modular incubator chamber (MP Biomedicals, Aurora, OH) at high oxygen saturation (95%) and incubated at 37°C. After 6 and 24 hours, biopsies were collected and total RNA was extracted for reverse-transcription PCR (RT-PCR) analysis.

### Cytokine induction in Caco-2 cells

Proinflammatory cytokine induction was performed by treating Caco-2 cells, in absence or in presence of bLf, with 5ng/mL of interferon gamma for 6 hours. Supernatants and cell extracts were processed for cytokines (IL-6, IL-8, and TNF-alpha) by quantitative RealTime-PCR.

### Real-time PCR

Expression of TNF-alpha, IL-8, and IL-6 was detected by Real-time PCR. Primers were designed to non-redundant sequences using Primer Express V3.0 (Applied Biosystems, Foster City, California. United States).

Primers were: TNF-alpha: fwd 5'-TCTGGCCCAGGCAGTCAGATC-3'; rvs 5'-CAGTGATGTTGGGGATAAAGAGC-3'; IL-8: fwd 5'-ATGACTTCCAAGCTGGCCGTGGCT-3'; rvs 5'-TCTCAGCCCTCTTCAAAAACTTCTC-3'; IL-6: fwd 5'-AGGGCTCTTCGGCAAATGTA3'; rvs 5'-GAAGGAATGCCCATTAACAACAA-3'.

Total RNA (1µg) was reverse-transcribed to cDNA by a High Capacity cDNA Reverse Transcription Kit (Applied Biosystems). Real-time PCR amplification was done with an ABI®PRISM 7300 Sequence Detection System, using the SYBR®Green kit (Applied Biosystems). Relative transcript levels were determined using GAPDH as the endogenous control genes: primers used: 5'- TCATCAATGGAAATCCCATCA-3 and 5'- GCCAGCATCGCCCCACTT-3'.

### Results

### Effect of lactoferrin and its pepsin hydrolysate on LF82 adhesion to Caco-2 cells

A preliminary set of experiments was carried out in order to determine the maximal non-cytotoxic and non-bactericidal concentration of lactoferrin and its pepsin hydrolysate. For this purpose, 2-fold serial dilutions of proteins from 4 mg/ml in MEM were incubated with Caco-2 cells for 24h at 37°C. Under these conditions, up to 4 mg/ml protein did not affect any of the cytotoxicity parameters (data not shown). The antibacterial activity of lactoferrin and its pepsin hydrolysate was then investigated and results obtained showed that all proteins, up to 40 mg/ml, did not affect bacterial cells viability. The effect of 1 mg/ml proteins on LF82 adhesion to Caco-2 cells was then tested.

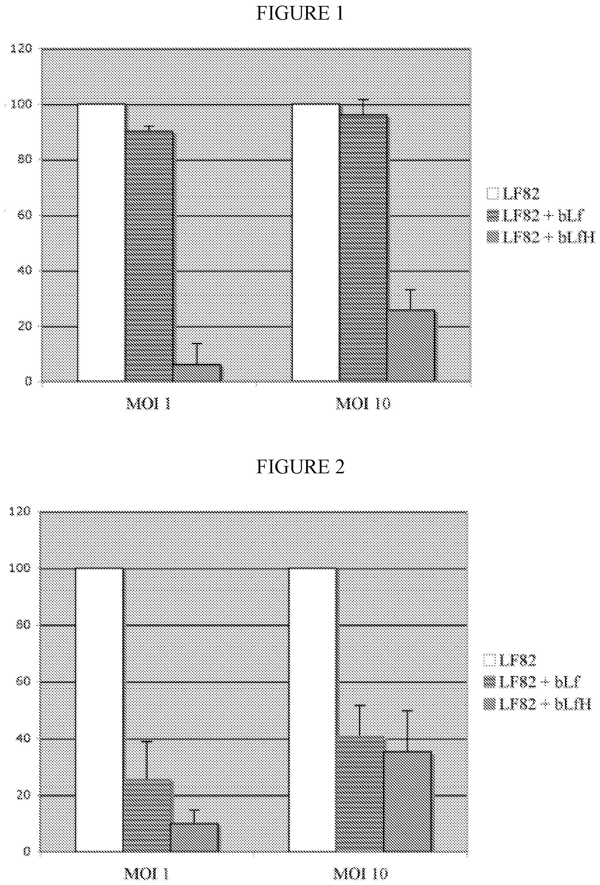

As shown in Figure 1, LF82 attachment to Caco-2 cell membranes was not significantly affected by bLf whereas bLfH treatment inhibited bacterial adhesion of about 95 and 75% with MOI 1 and MOI 10, respectively.

### Effect of lactoferrin and its pepsin hydrolysate on LF82 invasion into Caco-2 cells

The effect of bLf and bLfH on LF82 invasion was then tested. As shown in Figure 2, LF82 internalisation into Caco-2 cells was significantly inhibited by both bLf and bLfH, being bLfH more active than bLf. In fact bLfH treatment resulted in an inhibition of bacterial invasion of about 90 and 65% with MOI 1 and MOI 10, respectively, whereas, in the same experimental conditions, bLf reduced LF82 entry of about 75 and 60%.

### Effect of bLf and bLfH on pro-inflammatory cytokine expression

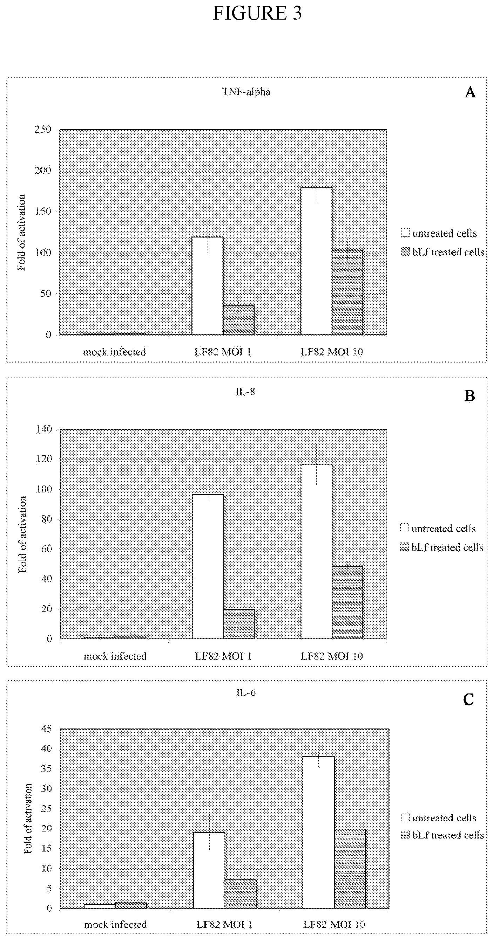

In order to understand whether bLf and bLfH were able to influence the production of pro-inflammatory cytokines and, thus, to modulate inflammation, mRNA expression of tumor necrosis factor-alpha (TNF-alpha), interleukin 8 (IL-8), and interleukin 6 (IL-6) was analysed in Caco-2 cells infected or not with LF82. The infection of monolayers was performed in the absence or presence of bLf or bLfH as described for the adhesion test. In these experimental conditions, the absence of antibacterial activity of bLf and bLfH was relevant to exclude that different cytokine mRNA expression could be related to a different number of viable adherent bacteria. Results obtained showed that the addition of bLf did not influence pro-inflammatory cytokines m-RNA expression in mock-infected cells whereas significantly decreased the TNF-alpha, IL-8, and IL-6 mRNA expression in cells infected with LF82 (Figure 3). This effect was not due to a direct action on bacterial invasion as bLfH, although capable to prevent bacterial internalization, was unable to down regulate pro-inflammatory cytokines m-RNA expression (data not shown).

### Effect of bLf on TNF-alpha, IL-8, and IL-6 mRNA expression in cultured mucosal explants infected with LF82

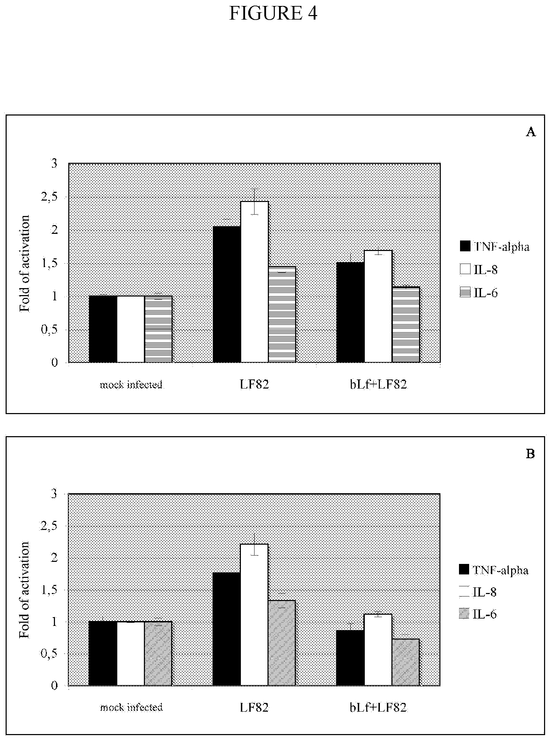

To emulate the in vivo conditions, the effect of bLf on pro-inflammatory cytokine m- RNA expression was also analyzed in the biopsies taken from two CD subjects as described in Materials and methods, which were infected with LF82 and cultured for 6 and 24 hours as described in Materials and Methods. Similar to in vitro experiments, the results obtained showed that the addition of bLf decreased the TNF-alpha, IL-8, and IL-6 mRNA expression in cultured mucosal explants infected with LF82, this inhibition being more evident after 24 hours (Figure 4).

### Effect of bLf on pro-inflammatory cytokine m-RNA expression in Caco-2 cells treated with INF-gamma

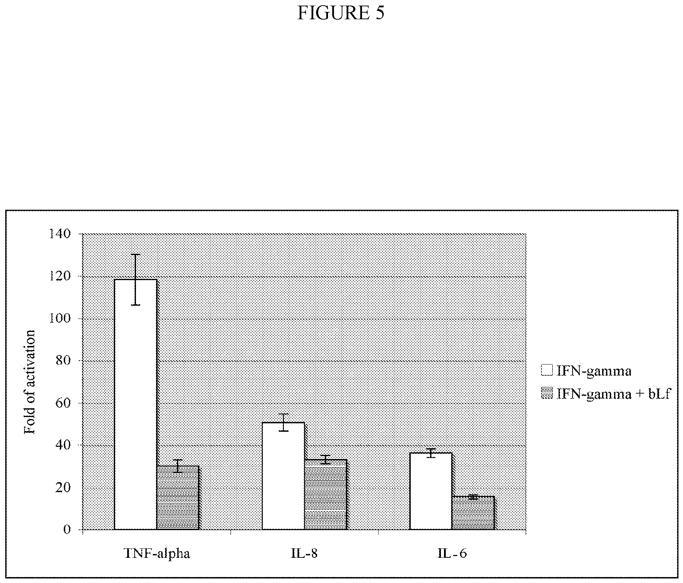

Lactoferrin expression is upregulated in response to inflammatory stimuli. The anti-inflammatory activity occurs through inhibition of binding of lipopolysaccharide endotoxin to inflammatory cells, as well as through interaction with epithelial cells at local sites of inflammation to inhibit inflammatory cytokine production (Conneely OM. Antiinflammatory activities of lactoferrin. J Am Coll Nutr. 2001 20:389S-395S.). To evaluate a possible direct effect of bLf on pro-inflammatory cytokine synthesis, Caco-2 cells were treated with INF-gamma for 6 hours in presence or absence of bLf. Results obtained showed that the addition of bLf decreased the pro-inflammatory cytokine mRNA expression, in particular TNF-alpha, in cells treated with INF-gamma (Figure 5).

The following non limiting examples are intended to explain the invention in greater detail.

### EXAMPLE 1.

200 g of bovine lactoferrin are loaded in a high shear mixer equipped with heating jacket and are kneaded with 50 g of cetyl alcohol previously heated to the softening temperature.

Then, the mixture is granulated with an aqueous suspension containing 15 g of polyvinylpyrrolidone until a homogeneous granulated product is obtained.

5 g of polyvinylpyrrolidone and 80 g of hydroxypropylmethylcellulose hydrophilic matrix are added in the same high share mixer.

The components are mixed until homogeneous dispersion of the matrices. Then 100 g of microcrystalline cellulose, 5 g of magnesium stearate, 5 g of talc and 10 g of colloidal silica are added in the order of mention.

The mixture is compressed so as to obtain a final unitary weight of 470 mg/tablet, suitable to provide 200 mg of active ingredient for each tablet.

The resulting tablets are then film coated with hydroxypropylmethylcellulose and plasticizers.

The tablets subjected to dissolution test in gastric juice and in simulated intestinal environment according to the current edition of European Pharmacopea showed the following release profile: after 60 minutes less than 30%, after 180 minutes is less than 60%, after 5 hours less than 80%.

### EXAMPLE 2.

20 g of bovine lactoferrin are loaded in a granulator/homogenizer and 10 g of carnauba wax and 45 g of cetyl alcohol are added. The mixture is heated to the softening temperature and a homogeneous mixture is obtained.

155 g of hydroxypropyl methylcellulose hydrophilic matrix and 50 mg of polycarbophil are added in the same mixer.

The components are mixed until an homogeneous dispersion of the matrices is obtained. Then, 210 g of calcium phosphate, 5 g of magnesium stearate and 5 g of colloidal silica are added in the order of mention.

The mixture is compressed up to a final unitary weight of 500 mg / tablet suitable to provide 20 mg of active ingredient for each tablet.

The resulting tablets are then film coated with hydroxypropylmethylcellulose and plasticizers.

The tablets subjected to dissolution test in gastric juice and in simulated intestinal environment according to the current edition of European Farmacopea showed the following release profile: after 60 minutes less than 25%, after 180 minutes less than 50%, after 5 hours less than 70%.

### EXAMPLE 3.

300 g microgranules made of microcrystalline cellulose and corn starch, are loaded in a Wuster fluid bed system; 100 g of bovine lactoferrin with 20 g of polyvinylpyrrolidone are suspended at the same time by being loaded over the microgranules.

Film coating is further performed by an aqueous solution containing 50 g polyvinylpyrrolidone and 50 g of ethylcellulose to obtain homogeneous microgranules.

Then capsules of size 0 are filled with the mixture to a final unitary weight of 520 mg/capsule to obtain 100 mg of active ingredient per single capsule.

The capsules subjected to dissolution test in gastric juice and in simulated intestinal environment according to the current edition of European Farmacopea showed the following release profile: after 120 minutes in gastric juices 0%, after 60 minutes in enteric juice less than 25%, after 180 minutes less than 50%, after 6 hours less than 80%.

### EXAMPLE 4.

200 g of microcrystalline cellulose with 100 g of corn starch are loaded in a fluidized bed with 200 g of bovine Lactoferrin.

The mixture is granulated with an aqueous solution containing 50 g polyvinylpyrrolidone to obtain a homogeneous granulated product.

Then, 150 g of hydroxypropyl methylcellulose, 5 g of magnesium stearate and 5 g of colloidal silica are added.

The mixture is then compressed to a final unitary weight up of 710 mg/tablet to provide single tablets containing 200 mg of active ingredient.

The resulting tablets are then film coated with polymethacrylates and plasticizers to ensure gastric resistance and prevent the release of active ingredient in the stomach.

The tablets subjected to dissolution test in gastric juice and in simulated intestinal environment showed the following release profile: after 120 minutes in gastric juices 0%, after 60 minutes in enteric juice less than 25%, after 180 minutes less than 50%, after 6 hours less 80%.

### EXAMPLE 5.

800 g of bovine lactoferrin are loaded in a granulator / homogenizer, and 400 g of hydroxypropylmethylcellulose, 480 g of mannitol, 252 g of microcrystalline cellulose are added.

The mixture was stirred for 15 minutes.

20 g stearic acid, 8 g of croscarmellose, 20 g of lecithin, 40 g of colloidal silica and 20 g of magnesium stearate were added in the order of mention.

The mixture was compressed to a final unitary weight of 510 mg / tablet to provide single tablets containing each 200 mg of active ingredient.

The obtained tablets are coated with 500 g of shellac (aquagold 25%), 50 g of hydroxypropyl methylcellulose 25 g of glycerine to obtain a 50 mg enteric film coated tablet.

The coated tablets were subjected to dissolution test gastric juice and in simulated intestinal environment according to the current edition of European Farmacopea. The tests showed the following release profile: after 120 minutes in gastric juices 0%, after 60 minutes in enteric juice less than 25%, after 180 minutes less than 50% after 8 hours no less 80% after 12 hours> 80%.

### EXAMPLE 6

800 g of bovine Lactoferrin are loaded in a mixer/homogenizer, then 400 g of hydroxypropylmethylcellulose, 480 g of mannitol, 252 g of microcrystalline cellulose are added.

The mixture was stirred for 15 minutes to obtain a homogeneous mixture.

Are added in the order 20 g stearic acid, 8 g of croscarmellose, 20 g of lecithin, 40 g of colloidal silica and 20 g of magnesium stearate.

The mixture was compressed to a final unitary weight of 510 mg/tablet to provide tablets containing 200 mg of active ingredient per single tablet.

The tablets obtained are coated with an aqueous solution containing 40 g of hydroxypropylmethylcellulose 8 g of talc to obtain 12 mg film coated tablet.

The tablets subjected to dissolution test gastric juice and in simulated intestinal environment according to the current edition of European Farmacopea showed the following release profile: after 60 minutes less than 25%, after 180 minutes less than 60%, after 8 hours less than 80.

### EXAMPLE 7.

800 g of bovine Lactoferrin were loaded in a mixer / homogenizer and 416 g of microcrystalline cellulose, 496 g of mannitol are added.

The mixture was stirred for 15 minutes. 20 g stearic acid, 160 mg crosslinked PVP, 60 g of croscarmellose, 20 g of lecithin, 40 g of colloidal silica and 20 g of magnesium stearate are added in the mentioned order.

The mixture was compressed up to a final unitary weight of 510 mg / tablet to provide tablets containing 200 mg of active ingredient per single tablet.

The tablets obtained were coated with 480 g of shellac (aquagold 25%), 48 g of hydroxypropyl methylcellulose 24 g of glycerine to get 50 mg film enteric coated tablet.

The coated tablets subjected to dissolution test gastric juice and in simulated intestinal environment according to the current edition of European Farmacopea showed the following release profile: after 120 minutes in gastric juices 0%, after 60 minutes in enteric juice less than 25%, after 180 minutes> 70 % after 300 minutes> 80 %.

### EXAMPLE 8

200 g of bovine lactoferrin were loaded in high share mixer with heating jacket and kneaded with 50 g of Lauroyl polyoxyglycerides previously brought to the softening temperature.

The mixture was further granulated with a solution/aqueous suspension containing 15 g of polyvinylpyrrolidone until a homogeneous granulate was obtained.

5 g of crospovidone and 80 g of hydroxypropylmethylcellulose were added in the same mixer.

The components were mixed until a homogeneous dispersion of the matrices was obtained. Then, 100 g of microcrystalline cellulose, 5 g of magnesium stearate, 5 g of talc and 10 g of colloidal silica were added in the order of mention.

The mixture was compressed up to a final unitary weight of 470 mg / cpr that is suitable for administering 200 mg of active ingredient for each tablet.

The resulting tablets were then film coated with hydroxypropylmethylcellulose and plasticizers.

The tablets, subjected to dissolution test gastric juice and in simulated intestinal environment according to the current edition of European Farmacopea, showed the following release profile: after 60 minutes no more than 30%, after 180 minutes is not more than 60%, after 5 hours no more of 80%.

### EXAMPLE 9

20 g of bovine lactoferrin were loaded in a granulator / homogenizer and 55 g of stearoyl glycerides were then added. The mixture was granulated up to the softening temperature, to obtain a homogeneous mixture. 205 g of hydroxypropyl methylcellulose were then added in the same mixer.

The components were mixed until homogeneous dispersion of the matrices and then 210 g of calcium phosphate, 5 g of magnesium stearate and 5 g of colloidal silica were added in the order of mention.

The mixture was compressed up to a final unitary weight of 500 mg/cpr suitable for administering 20 mg of active ingredient for each tablet.

The resulting tablets were then film coated with hydroxypropylmethylcellulose and plasticizers.

The tablets subjected to dissolution test gastric juice and in simulated intestinal environment according to the current edition of European Farmacopea showed the following release profile: after 60 minutes no more than 25%, after 180 minutes is not more than 50%, after 5 hours no more of 70%.

## Claims

1. Oral formulation providing modified release dosage comprising lactoferrin for use in the treatment of inflammatory bowel disease associated with Adherent-invasive Escherichia coli (AIEC) invasion.

2. Oral formulation for use according to claim 1, wherein said inflammatory bowel disease is Crohn's disease.

3. Oral formulation for use according to any of the previous claims, comprising lactoferrin dissolved and/or dispersed and/or embedded in a matrix and an outer coating, wherein said matrix comprises:
- substances selected in the group of: lipid substances, hydrophilic substances, amphiphilic substances and mixtures thereof;
- and optionally pharmaceutically acceptable excipients.

4. Oral formulation for use according to claim 3, wherein said lipid substances are selected in the group consisting of fatty alcohols, fatty acids, triglycerides, waxes.

5. Oral formulation for use according to claim 3, wherein said hydrophilic substances are selected in the group consisting of polymers or copolymers of acrylic or methacrylic acid, alkyl vinyl ether polymers, hydroxyalkylcelluloses, carboxyalkylcelluloses, polysaccharides, alginates, pectins, starches, natural and synthetic gums, polycarbophil and mixtures thereof.

6. Oral formulation for use according to claim 3, wherein said amphiphilic substances are selected in the group consisting of lecithin, phosphatidylcholine, phosphatidyl- ethanolamine, glycol alkyl ethers such as diethylene glycol monomethyl ether, macrogolglycerides, polyethylene glycol hydroxystearates, waxes, sodium laurylsulfate, sodium dodecylsulfate, polysorbates, cholic acids, poloxamer, sodium sulphosuccinate, sodium laurylsarcosinate and mixtures thereof.

7. Oral formulation for use according to claim 1, wherein lactoferrin is dissolved and/or dispersed and/or embedded in a reservoir core that is coated by a semipermeable film.

8. Oral formulation for use according to any of the previous claims, further comprising gastroresistant coating agents selected in the group consisting of Shellac, Polymethacrylates, Cellulose Acetophtalate, Alginate Derivatives.

9. Oral formulation dosage for use according to any of the previous claims, **characterized in that** the amount of lactoferrin is between 50 mg and 250 mg.

## Patentansprüche

1. Orale Formulierung für eine modifizierte Abgabedosierung, enthaltend Laktoferrin, zur Anwendung bei der Behandlung einer entzündlichen Darmerkrankung, die von einem kontakt-invasiven Befall von Escherichia-coli-Bakterien (AIEC) begleitet ist.

2. Orale Formulierung zur Anwendung gemäß Anspruch 1, wobei die besagte entzündlichen Darmerkrankung Morbus Crohn ist.

3. Orale Formulierung zur Anwendung gemäß einem der vorangehenden Ansprüche, umfassend Laktoferrin, gelöst und/oder dispergiert und/oder eingebettet in einer Matrix und einem äußeren Überzug, wobei die besagte Matrix umfasst:
- Substanzen, ausgewählt aus der Gruppe von: Lipid-Substanzen, hydrophilen Substanzen, amphiphilen Substanzen und Mischungen daraus;
- sowie optional pharmazeutisch zulässigen Hilfsstoffen.

4. Orale Formulierung zur Anwendung gemäß Anspruch 3, wobei die besagten Lipid-Substanzen ausgewählt sind aus der Gruppe umfassend Fettalkohole, Fettsäuren, Triglyceride, Wachse.

5. Orale Formulierung zur Anwendung gemäß Anspruch 3, wobei die besagten hydrophilen Substanzen ausgewählt sind aus der Gruppe umfassend Polymere oder Copolymere der Acrylsäure oder Methacrylsäure, Alkylvinyletherpolymere, Hydroxyalkylzellulosen, Carboxyalkylzellulosen, Polysaccharide, Alignate, Pektine, Stärken, natürliche und synthetische Harze, Polycarbophil und Mischungen daraus.

6. Orale Formulierung zur Anwendung gemäß Anspruch 3, wobei die besagten amphiphilen Substanzen ausgewählt sind aus der Gruppe umfassend Lecithin, Phosphatidylcholin, Phosphatidylethanolamin, Glycolalkylether wie Diethylenglycolmonomethylether, Makrogolglyceride, Polyethylenglycolhydroxystearate, Wachse, Natriumlaurylsulfat, Natriumdodecylsulfat, Polysorbate, Cholsäure, Poloxamer, Natriumsulfosuccinat, Natriumlaurylsarkosinat und Mischungen daraus.

7. Orale Formulierung zur Anwendung gemäß Anspruch 1, wobei Laktoferrin gelöst und/oder dispergiert und/oder eingebettet ist in einem Speicherkern, der von einem halbdurchlässigen Film überzogen ist.

8. Orale Formulierung zur Anwendung gemäß einem der vorangehenden Ansprüche, ferner umfassend magensaftresistente Überzugsmittel, ausgewählt aus der Gruppe umfassend Schellack, Polymethacrylate, Zellulose-Acetophtalat, Alginat-Derivate.

9. Orale Formulierung zur Anwendung gemäß einem der vorangehenden Ansprüche, **dadurch gekennzeichnet, dass** die Menge von Laktoferrin zwischen 50 mg und 250 mg liegt.

## Revendications

1. Formulation à usage oral permettant un dosage à libération modifiée comprenant de la lactoferrine pour une utilisation dans le traitement d'une maladie intestinale inflammatoire associée à une invasion par Escherichia coli invasive adhérente (AIEC).

2. Formulation à usage oral pour une utilisation selon la revendication 1, dans laquelle ladite maladie intestinale inflammatoire est la maladie de Crohn.

3. Formulation à usage oral pour une utilisation selon l'une quelconque des revendications précédentes, comprenant de la lactoferrine dissoute et/ou dispersée et/ou noyée dans une matrice et un enrobage extérieur, dans laquelle ladite matrice comprend :
- des substances choisies dans le groupe comprenant les substances lipidiques, les substances hydrophiles, les substances amphiphiles et leurs mélanges ;
- et éventuellement des excipients pharmaceutiquement acceptables.

4. Formulation à usage oral pour une utilisation selon la revendication 3, dans laquelle lesdites substances lipidiques sont choisies dans le groupe constitué par les alcools gras, les acides gras, les triglycérides, les cires.

5. Formulation à usage oral pour une utilisation selon la revendication 3, dans laquelle lesdites substances hydrophiles sont choisies dans le groupe constitué par les polymères ou copolymères d'acide acrylique ou méthacrylique, les polymères d'alkylvinyléther, les hydroxyalkylcelluloses, les carboxyalkylcelluloses, les polysaccharides, les alginates, les pectines, les amidons, les gommes naturelles et synthétiques, le polycarbophile, et leurs mélanges.

6. Formulation à usage oral pour une utilisation selon la revendication 3, dans laquelle lesdites substances amphiphiles sont choisies dans le groupe constitué par la lécithine, la phosphatidylcholine, la phosphatidyl-éthanolamine, les éthers alkyliques de glycol tels que l'éther monométhylique de diéthylèneglycol, les macrogol-glycérides, les hydroxystéarates de polyéthylèneglycol, les cires, le laurylsulfate de sodium, le dodécylsulfate de sodium, les polysorbates, les acides choliques, un poloxamère, le sulfosuccinate de sodium, le laurylsarcosinate de sodium et leurs mélanges.

7. Formulation à usage oral pour une utilisation selon la revendication 1, dans laquelle la lactoferrine est dissoute et/ou dispersée et/ou noyée dans un cœur de réservoir qui est enrobé d'un film semi-perméable.

8. Formulation à usage oral pour une utilisation selon l'une quelconque des revendications précédentes, comprenant en outre des agents d'enrobage gastrorésistants choisis dans le groupe constitué par la gomme laque, les polyméthacrylates, l'acétophtalate de cellulose, les dérivés d'alginate.

9. Formulation à usage oral pour une utilisation selon l'une quelconque des revendications précédentes, **caractérisée en ce que** la quantité de lactoferrine est comprise entre 50 mg et 250 mg.


### SEQUENCE LISTING

<110> Giellepi S.p.A.
<120> LACTOFERRIN FOR THE TREATMENT OF IBD ASSOCIATED WITH BACTERIAL INVASION
<130> BW697M
<160> 8
<170> BiSSAP 1.2
<210> 1
   <211> 21
   <212> DNA
   <213> Artificial Sequence
<220>
   <221> source
   <222> 1..21
   <223> /mol_type="unassigned DNA" /note="TNF-alpha: fwd " /organism="Artificial Sequence"
<400> 1
   tctggcccag gcagtcagat c 21
<210> 2
   <211> 23
   <212> DNA
   <213> Artificial Sequence
<220>
   <221> source
   <222> 1..23
   <223> /mol_type="unassigned DNA" /note="TNF-alpha: rvs" /organism="Artificial Sequence"
<400> 2
   cagtgatgtt ggggataaag agc 23
<210> 3
   <211> 24
   <212> DNA
   <213> Artificial Sequence
<220>
   <221> source
   <222> 1..24
   <223> /mol_type="unassigned DNA" /note="IL-8: fwd " /organism="Artificial Sequence"
<400> 3
   atgacttcca agctggccgt ggct 24
<210> 4
   <211> 25
   <212> DNA
   <213> Artificial Sequence
<220>
   <221> source
   <222> 1..25
   <223> /mol_type="unassigned DNA" /note="IL-8: rvs" /organism="Artificial Sequence"
<400> 4
   tctcagccct cttcaaaaac ttctc 25
<210> 5
   <211> 20
   <212> DNA
   <213> Artificial Sequence
<220>
   <221> source
   <222> 1..20
   <223> /mol_type="unassigned DNA" /note="IL-6: fwd" /organism="Artificial Sequence"
<400> 5
   agggctcttc ggcaaatgta 20
<210> 6
   <211> 23
   <212> DNA
   <213> Artificial Sequence
<220>
   <221> source
   <222> 1..23
   <223> /mol_type="unassigned DNA" /note="IL-6: Rev" /organism="Artificial Sequence"
<400> 6
   gaaggaatgc ccattaacaa caa 23
<210> 7
   <211> 21
   <212> DNA
   <213> Artificial Sequence
<220>
   <221> source
   <222> 1..21
   <223> /mol_type="unassigned DNA" /note="GAPDH primers For" /organism="Artificial Sequence"
<400> 7
   tcatcaatgg aaatcccatc a 21
<210> 8
   <211> 18
   <212> DNA
   <213> Artificial Sequence
<220>
   <221> source
   <222> 1..18
   <223> /mol_type="unassigned DNA" /note="GAPDH primer rev" /organism="Artificial Sequence"
<400> 8
   gccagcatcg ccccactt 18